# EUROPÄISCHE PATENTSCHRIFT

(11) **EP 2 484 318 B1**
(45) Veröffentlichungstag und Bekanntmachung des Hinweises auf die Patenterteilung: **21.05.2014**
(21) Anmeldenummer: 12153205.5
(22) Anmeldetag: 31.01.2012
(51) Int. Cl.: A61F 9/02, A42B 3/18

(54) **Sportbrille**
Sports glasses
Lunette de sport

(30) Priorität: 03.02.2011 DE 202011000263 U
(43) Veröffentlichungstag der Anmeldung: 08.08.2012
(73) Patentinhaber: CASCO Group Societate in Comandita Simpla (SCS), Satu Mare 440247 (RO)
(72) Erfinder: Krauter, Markus, 73635 Rudersberg (DE)
(74) Vertreter: Kailuweit, Frank

(56) Entgegenhaltungen:
- EP-A1- 2 305 053
- DE-U1- 20 306 048
- US-A1- 2005 183 190
- US-A1- 2009 300 830

## Beschreibung

Die Erfindung betrifft eine Sportbrille mit einem Sporthelm.

Bei vielen Sportarten, wie z. B. beim Radfahren, beim Abfahrtslauf, beim Skispringen oder Snowboarden werden aus Sicherheitsgründen Sporthelme und Sicherheitsbrillen getragen.

Bekannte Sportbrillen weisen entweder Bügel oder ein breites Gummiband zur Fixierung am Kopf des Trägers oder auf dem Umfang des Helmes auf. Bügelsysteme sind zur Benutzung mit einem Sporthelm ungeeignet, da die Bügel unter dem Helm zu unangenehmen Druckstellen führen können. Sportbrillen mit einem breiten Gummiband zur Befestigung sind aus dem Skisport bekannt. Nachteilig ist, dass das Gummiband leicht vom Helm abrutschen kann. Zudem ist nur eine axiale Einstellbarkeit der Länge des Gummibandes möglich.

Beispielsweise wird in der DE 203 06 048 U1 eine Gesamtheit aus Brille und Helm vorgeschlagen, wobei die Helmschale auf jeder ihrer seitlichen Ränder eine Haltebefestigung aufweist, die vorgesehen ist, die elastische Verbindung zwischen Helm und Brille mindestens in der oberen Position zu halten. Nachteilig an dieser Lösung ist die schlechte Bedienbarkeit insbesondere mit Handschuhen.

Die US 4,918,753 A hat eine Brille zum Gegenstand, die mittels seitlich am Brillenrahmen befestigter, elastischer Bänder an einem pilzförmigen Befestigungsteil seitlich an der Helmschale befestigt wird. Auch diese Lösung ist insbesondere mit Handschuhen schlecht bedienbar. Zudem wird die Brille durch das breite Band nur unzureichend am Gesicht des Trägers befestigt.

Aufgabe der vorliegenden Erfindung ist es, eine Sportbrille mit einem Sporthelm vorzuschlagen, die einen verbesserten Tragekomfort bietet und kostengünstig herzustellen ist.

Die Sportbrille zur Verwendung mit einem Sporthelm weist einen Rahmen mit einer gewölbten oder planen Sichtscheibe auf. In einer alternativen Gestaltung nimmt der Rahmen zwei plane oder gewölbte Brillengläser auf. Dabei kann an einer Scheibe außen oder innen eine zweite Scheibe beanstandet aufgebracht sein. In einer anderen Ausführung werden einzelne Sichtscheiben beabstandet in den Rahmen eingesetzt.

Die Sportbrille weist Befestigungsschnüre auf, die mit Befestigungselementen verbunden sind. Die Befestigungselemente können dabei zur Befestigung der Brille an einem Helm mit in der Helmschale verankerten Befestigungsmitteln korrespondieren, oder auch als dehnbares Befestigungselement, insbesondere Gummiband, ausgeführt sein.

Die Befestigungsmittel und Befestigungselemente können als paarweise Dauermagneten ausgebildet sein wie in der EP 2 305 053 A1 offenbart (Stand der Technik gemäß Artikel 54(3) EPÜ). Ein vorteilhaft als Knopf oder Hebel ausgebildeter Dauermagnet ist als Befestigungselement mit den Befestigungsschnüren der Sportbrille verbunden. Der zweite, vorzugsweise topfförmige Dauermagnet ist mit der Helmschale verbunden. Vorzugsweise ist dieser Magnet in einer Nut oder Führung an oder in der Helmschale verschiebbar und fixierbar angeordnet, um die Vorspannung der Befestigungsschnüre individuell einstellen zu können. Die Verwendung der Dauermagneten erleichtert durch die Selbstzentrierung bei der Annäherung der beiden paarigen Dauermagneten die Adaption der Sportbrille mit einem Sporthelm.

In einer bevorzugten Ausführungsform sind die beiden Enden einer Befestigungsschnur mit der Brille verbunden wie in der EP 2 305 053 A1 offenbart (Stand der Technik gemäß Artikel 54(3) EPÜ). In der dabei gebildeten Schlaufe ist ein Befestigungselement, aufweisend einen Dauermagneten, angeordnet. Im seitlichen Bereich der Helmschale sind Befestigungsmittel ebenfalls in Form von Dauermagneten so angebracht, dass zwischen diesen und den Dauermagneten der Befestigungselemente eine Anziehungskraft wirkt. Damit braucht der Benutzer beim Aufsetzen der Brille die Befestigungselemente nur in die Nähe der Befestigungsmittel an der Helmschale zu bringen und die Verbindung erfolgt aufgrund der Magnetkräfte selbstständig.

Bevorzugt weist eine Paarung von Dauermagneten (Befestigungsmittel in Helmschale - Befestigungselement an Befestigungsschnur) in der Berührungsfläche eine korrespondierende Erhöhung bzw. Vertiefung auf wie in der EP 2 305 053 A1 offenbart (Stand der Technik gemäß Artikel 54(3) EPÜ). Durch diese zentriert sich die Magnetpaarung bei Annäherung selbstständig. Zudem wird ein sehr guter Halt der Magnetverbindung erreicht, da Kräfte in Verbindungsebene besser aufgenommen werden können. Bevorzugt hat die Erhöhung/Vertiefung in den Dauermagneten eine kreiszylindrische Form, kann aber auch die Form eines Kreiskegelstumpfes aufweisen. Somit wird eine kraft- und formschlüssige Verbindung erreicht.

In einer weiteren, gleichfalls bevorzugten Ausbildung weist das Befestigungsmittel einen Spannhebel auf, der an einer Drehachse des Befestigungsmittels dreh- oder schwenkbar gelagert ist. Der Spannhebel weist eine Bohrung oder einen offenen oder geschlossenen Kanal auf, der die schlaufenförmige Befestigungsschnur aufnimmt. Durch das Schwenken des Spannhebels wird die Vorspannung der Befestigungsschnur gelöst und die Sportbrille kann leicht abgenommen werden. Beim Anlegen der Sportbrille wird durch das Schwenken des Spannhebels in entgegengesetzter Richtung die notwendige Vorspannung erzeugt, um einen sicheren Sitz der Brille auf dem Gesicht des Trägers zu ermöglichen. Die Feinjustierung und Einstellung der Spannung der Befestigungsschnüre erfolgt vorteilhaft durch das bereits beschriebene, axial verschiebbare Spannelement.

In einer bevorzugten Ausführung hat der Sporthelm in den beiden Schläfenbereichen mehrere beabstandete Befestigungsmittel. Dadurch kann die Position der Brille (Neigungswinkel gegenüber der Senkrechten) auf dem Gesicht des Benutzers variiert werden.

In einer weiteren, gleichfalls bevorzugten Ausgestaltung können die selbstklebenden Befestigungsmittel (Dauermagneten) nachträglich vom Nutzer individuell auf der Helmschale angebracht werden.

In einer gleichfalls bevorzugten Ausgestaltung weist der Sporthelm an oder in der Helmschale in den Seitenbereichen, vorzugsweise im Schläfenbereich, eine oder mehrere Nuten oder Führungen auf, in denen die Befestigungsmittel (der Dauermagnet) axial verschiebbar und form- und/oder kraftschlüssig fixierbar sind. Dadurch kann die Vorspannung der Befestigungsschnüre mit einfachen Mitteln individuell eingestellt werden.

In einer anderen bevorzugten Ausführungsvariante sind beide Befestigungsschnüre der Brille mit einem dehnbaren Befestigungselement, insbesondere einem Gummiband, verbunden. Dessen Gesamtlänge ist im ungedehnten Zustand kleiner als der Umfang des Sporthelms, bzw. des Kopfes des Trägers. Durch Dehnung kann es seine Länge derart verändern, dass es im gedehnten Zustand um den Sporthelm oder auch direkt um den Kopf des Trägers umgelegt werden kann. Aufgrund der Elastizität ist das Befestigungselement ohne äußere Krafteinwirkung bestrebt in den ungedehnten Ausgangszustand zurückzukehren und bewirkt so eine Anpresskraft an den Sporthelm bzw. Kopf des Trägers. Das dehnbare Befestigungselement bzw. das Gummiband hat an seinen Enden Schlaufen, durch die die Befestigungsschnüre geführt werden.

In der bevorzugten Ausführungsform sind die Befestigungsschnüre mittels Scheibenverbindern durch Öffnungen in der Sichtscheibe bzw. den Sichtscheibensegmenten hindurch im Rahmen befestigt. Vorteilhaft wird die Sichtscheibe bzw. die Sichtscheibensegmente durch die Scheibenverbinder an den Rahmen angedrückt. Somit wird eine einfache Befestigung der Sichtscheibe am Rahmen realisiert. Die Scheibenverbinder weisen dabei einen Bereich auf, mit dem diese an der Sichtscheibe befestigt werden. Bevorzugt erfolgt die Verbindung von Scheibenverbindern und Sichtscheibe mittels Renkverbindung (Bajonettverbindung).

In der bevorzugten Ausführungsform wird die Sichtscheibe mittels der Scheibenverbinder am Rahmen der Brille befestigt. Dabei werden die Scheibenverbinder durch entsprechende Öffnungen in der Sichtscheibe hindurch geführt und mit dem Rahmen verbunden, wobei die Sichtscheibe fest mit dem Rahmen verbunden wird. Es können ebenfalls mehrere Scheibensegmente auf diese Weise am Rahmen angebracht werden. Bevorzugt erfolgt die Verbindung von Scheibenverbindern und Rahmen mittels Renkverbindung (Bajonettverbindung). Vorteilhaft kann so mit einfachen Mitteln die Sichtscheibe bzw. die Segmente der Sichtscheibe mit dem Rahmen verbunden werden, wodurch die Scheibe(n) einfach auswechselbar sind. In einer besonders bevorzugten Ausführungsform ist auf der Sichtscheibe innen beanstandet eine weitere Scheibe aufgebracht, wobei nur der mittlere Bereich als Doppelscheibe ausgeführt ist und am Rand nur die äußere Scheibe vorhanden ist. Dieser Randbereich der äußeren Scheibe überdeckt im zusammengebauten Zustand den Rahmen der Brille, wobei die Scheibenverbinder durch Öffnungen in diesem Randbereich der Scheibe diese mit dem Rahmen verbinden. Vorteilhaft werden so der optische Eindruck der Brille verbessert, da die Sichtscheibe den gesamten Rahmen überdeckt und Windgeräusche minimiert, da ausschließlich die Sichtscheibe und die Scheibenverbinder dem Fahrtwind ausgesetzt sind.

Weiterhin bevorzugt weisen die Scheibenverbinder Fortsätze auf, die im in der Sichtscheibe befestigten Zustand vom Auflagepunkt beabstandet und weitestgehend parallel zur Oberfläche der Sichtscheibe führen.

Damit die Renkverbindung sich nicht während der Benutzung löst, setzen sich die Scheibenverbinder von der Renkverbindung aus weitestgehend parallel und beabstandet zur Sichtscheibe fort. Durch die in seitlicher Richtung wirkende Zugkraft bei der Benutzung kann sich die Renkverbindung nicht lösen, da die Scheibenverbinder sich nicht drehen können. Dies hat zudem den Vorteil, dass die Befestigungsschnüre geführt werden, und somit eine Beschädigung der Schnüre oder der Sichtscheibe durch das Aufliegen auf der Kante der Sichtscheibe verhindert wird. Des Weiteren wirkt der Fortsatz der Scheibenverbinder als Hebel und die Brille wird weitestgehend mit gleichem Anpressdruck an das Gesicht des Trägers angedrückt. Hierfür sind die Scheibenverbinder bevorzugt in den oberen und unteren seitlichen Bereichen der Sichtscheiben befestigt. Bevorzugt wird die Renkverbindung verbunden, wenn der Fortsatz zur Brillenmitte zeigt und durch Verdrehen gesichert. Alternativ besteht auch die Möglichkeit, dass die Fortsätze zum Einstecken nach oben oder unten zeigen.

Um auch in Verbindungsrichtung ein Herausgleiten der Renkverbindung zu verhindern, sind die verbindenden Teile der Renkverbindung bevorzugt nicht exakt formschlüssig. So müssen die Scheibenverbinder auch in Verbindungsrichtung mit leichtem Druck in die rahmenseitig dafür vorgesehenen Öffnungen eingeschnappt werden. Zur verschleißfreien Anwendung ist die Renk- und Schnappverbindung dafür ausreichend elastisch gestaltet. Vorteilhaft können sich die Renkverbindungen so nicht versehentlich lösen.

Bevorzugt ist ein Scheibenverbinder ein Rohr aus Plastik mit einem ausreichenden Durchmesser zur Aufnahme der Befestigungsschnüre. Ein Ende des Rohres ist um ca. 90° abgewinkelt, wobei der konkrete Winkel von der geometrischen Ausgestaltung der Sichtscheibe abhängt, und weist dort die Mittel für die Renkverbindung auf. Zum Renkverschluss hin verdickt sich der Durchmesser des Rohres etwas. Kostengünstig muss zur Montage nur die Schnüre durch den Fortsatz geschoben werden, um dann das aus dem Bereich für die Renkverbindung austretenden Ende im einfachsten Fall mit einem Knoten zu sichern. Der auf der Sichtscheibe aufliegende Teil des Scheibenverbinders ist größer als die Öffnung in der Sichtscheibe und drückt diese somit im befestigten Zustand an den Rahmen an.

Die erfindungsgemäße Befestigungsanordnung zur Befestigung einer Sportbrille an einem Sporthelm, bestehend aus mindestens einer elastischen Befestigungsschnur, an deren Enden Scheibenverbinder befestigt sind und einem Befestigungselement, aufweisend einen Dauermagneten und mehrere Haltestege zur Aufnahme einer Befestigungsschnüre.

Die Sportbrille weist an jeder Außenseite des Rahmens oder der Sichtscheibe mindestens eine, vorzugsweise zwei biegeschlaffe, elastische Befestigungsschnüre auf, mit denen die Brille am Helm bzw. Kopf des Trägers fixiert wird. Vorzugsweise sind die Befestigungsschnüre als Schlaufe ausgebildet, deren Enden an der Schutzbrille angeordnet sind und der Schlaufenradius mit einem Befestigungselement in Verbindung steht, das die form- und/oder kraftschlüssige Verbindung mit dem Helm, vorzugsweise mit der Helmaußenschale ermöglicht.

In einer bevorzugten Ausführungsform weist jede der schlaufenförmigen Befestigungsschnüre ein Spannelement auf, das verschiebbar auf den paarigen Befestigungsschnüren angeordnet ist. Durch Verschieben des Spannelementes in Richtung zur Brille werden die vom Befestigungsmittel zur Brille konvergent verlaufenden Segmente der jeweiligen Befestigungsschnur zusammengezogen und so die Spannung der Befestigungsschnur erhöht. Durch Verschieben des Spannelementes in Richtung des Befestigungselements wird die Spannung der Befestigungsschnur verringert. Auf diese Weise lässt sich der Andruck der Brille gegen das Gesicht des Trägers regulieren.

Vorzugsweise bestehen die Befestigungsschnüre aus elastischer Rundlitze, runder Gummilitze, Rundgummi, Gummikordel oder Gummigarn mit Polyester- oder Polyamid- Fasern. Vorteilhaft bestehen die Befestigungsschnüre aus Neoprenband, da Neopren keine Flüssigkeit absorbiert.

Weiterhin bevorzugt besteht die Sichtscheibe aus schlagzähem Kunststoff, vorzugsweise aus Polycarbonat. Dadurch wird die Widerstandskraft erhöht und das Risiko von gefährlicher Splitterbildung bei Stürzen z. B. beim Ski- oder Fahrradsport vermindert.

Durch die geringe Teilezahl und den konstruktiv einfachen Aufbau kann die Sportbrille kostengünstig gefertigt werden. Ein weiterer Vorteil der erfindungsgemäßen Sportbrille besteht in ihrer Stabilität, da durch Druckeinwirkung kein Bügelsystem beschädigt werden kann. Weitere Vorteile sind das extrem geringe Gewicht und die Möglichkeit der individuellen Anpassung der Lage der Sportbrille im Bereich des Gesichtsschädels mit Hilfe der flexiblen, schlaufenartig ausgebildeten Befestigungsschnüre und der Befestigungsmittel.

Ein besonderer Vorteil der Ausführungsform mit Dauermagneten als Befestigungselement besteht in der Möglichkeit, die Sportbrille beim Absetzen (z. B. beim Warten am Skilift) an nur einem Dauermagneten zu belassen. In dieser Position kann die Sportbrille nicht vom Helm abrutschen. Dadurch, dass sich die Sportbrille in einer hängenden Position im Schläfenbereich des Sporthelmes befindet, ist das Gesichtsfeld des Trägers frei. Zudem kann es nicht zu einem Beschlagen der Sportbrille durch die aufsteigende Atemluft des Trägers kommen. Durch die vorzugsweise verwendeten, selbstzentrierenden Befestigungsmittel kann die Sportbrille auch mit Handschuhen leicht und bequem wieder angelegt werden.

Durch die alternativen Befestigungsmittel Dauermagneten bzw. dehnbares Befestigungselement (Gummiband) ist eine Brille mit hohem Komfort sowohl mit Helm als auch ohne Helm (bzw. einem Helm ohne Magneten) nutzbar.

Die erfindungsgemäße Befestigungsanordnung weist ein dehnbares Befestigungselement, bevorzugt als Gummiband ausgeführt auf, an dessen Enden jeweils eine Befestigungsschnüre befestigt ist, wobei an den Enden der Befestigungsschnüre jeweils Scheibenverbinder angeordnet sind.

Die Verbindung zwischen dehnbarem Befestigungselement und Befestigungsschnüre kann dabei auf verschiedenste Weise erfolgen. Bevorzugt wird das Ende des Gummibandes zu einer Schlaufe/Kanal genäht, durch die die Befestigungsschnüre geführt sind.

Nachfolgend werden mehrere Ausführungsbeispiele der Erfindung beschrieben und anhand der Figuren erläutert.

Dabei zeigen:
- Figur 1:: eine Sportbrille mit einem Sporthelm mit einem, auf den schlaufenförmigen Befestigungsschnüren verschiebbaren Spannelement und einem, am Befestigungsmittel angeordneten Schwenkhebel,
- Figur 2:: die im befestigten Zustand dem Helm zugewandte Seite einer alternativen Ausgestaltung des Befestigungselements 6,
- Figur 3:: eine Sportbrille mit einem Befestigungselement entsprechend Figur 2,
- Figur 4:: einen Scheibenverbinder zur Befestigung der Befestigungsschnüre im Rahmen, in der Sichtscheibe oder durch Öffnungen in der Sichtscheibe hindurch im Rahmen und
- Figur 5:: eine alternative Ausgestaltung der Sportbrille mit einem schlaufenförmigen, dehnbaren Befestigungselement das mittels der Befestigungschnüre mit der Brille in Verbindung steht.

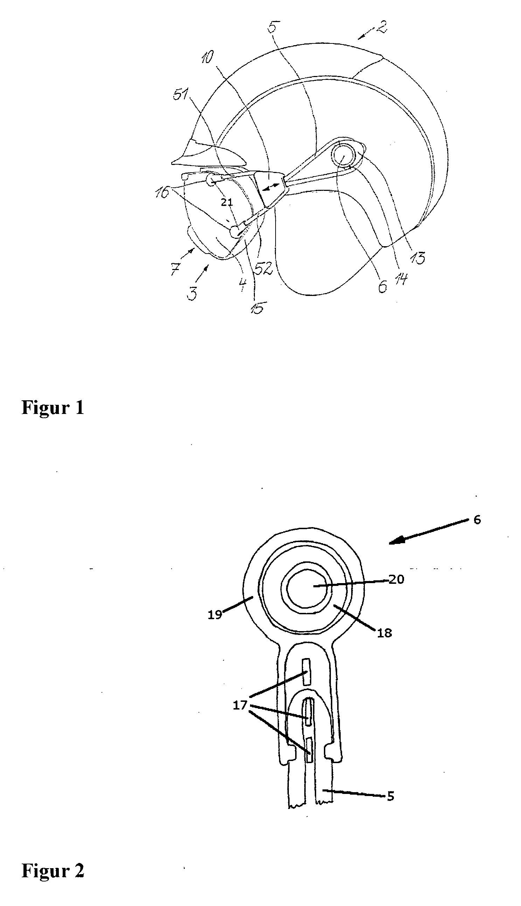

**Figur 1** zeigt eine Sportbrille 3 mit Rahmen 15. Am Rahmen 15 sind im Randbereich beidseitig schlaufenförmige Befestigungsschnüre 5 an zwei Anlagepunkten 16 mittels Scheibenverbinder 21 in der Sichtscheibe 4 angelenkt. Die Scheibenverbinder 21 sind mittels Renkverbindung (Bajonettverbindung) in der Sichtscheibe befestigt. Dabei ist sowohl am oberen Strang 51 der Befestigungsschnur 5 als auch am unteren Strang 52 der Befestigungsschnur 5 jeweils ein Scheibenverbinder 21 angeordnet. Die Scheibenverbinder 21 am Ende der beiden oberen Stränge 51 der Befestigungsschnüre 5 sind dabei im oberen seitlichen Bereich der Sichtscheibe befestigt und die Scheibenverbinder 21 an den Enden der beiden unteren Strängen 52 der Befestigungsschnüre 5 entsprechend im unteren seitlichen Bereich der Sichtscheibe 4. Dadurch wird die freie Sicht des Benutzers durch die Scheibenverbinder 21 nicht eingeschränkt.

Die Scheibenverbinder 21 sind wie oben beschrieben mittels Renkverbindung (Bajonettverbindung) in der Sichtscheibe 4 befestigt, wobei diese zur Mitte der Sichtscheibe 4 gerichtet eingesetzt werden und dann durch die Drehung in Richtung der Scheibenaußenseite in der Sichtscheibe 4 befestigt sind. Die Scheibenverbinder 21 sind somit bei der Benutzung drehbar und passen sich dadurch an das Gesicht und die Kopfform des Trägers an. Von der Befestigung in der Sichtscheibe 4 setzt sich der Scheibenverbinder 21 vom Anlagepunkt 16 noch ca. 2 cm beabstandet und weitestgehend parallel zu Sichtscheibe fort.

Das andere, schlaufenförmige Ende der Befestigungsschnur 5 steht mit einem Spannhebel 13 in Verbindung, der schwenkbar am Befestigungselement 6 angeordnet ist.

Der Spannhebel 13 weist einen geschlossenen Kanal auf, der die schlaufenförmige Befestigungsschnur 5 aufnimmt. Durch das Schwenken des Spannhebels 13 aus der dargestellten Position in Richtung der Sportbrille 3 wird die Vorspannung der Befestigungsschnur 5 gelöst und die Sportbrille 3 kann leicht abgenommen werden. Beim Anlegen der Sportbrille 3 wird durch das Schwenken des Spannhebels 13 in entgegengesetzter Richtung die notwendige Vorspannung erzeugt, um einen sicheren Sitz der Brille auf dem Gesicht des Trägers zu ermöglichen. Die Feinjustierung und Einstellung der Spannung der Befestigungsschnüre 5 erfolgt durch das bereits axial verschiebbare Spannelement 10. Das Spannelement 10 ist als Kunststoff-Spritzgussteil ausgebildet. Im trapezförmigen Gehäuse des Spannelementes sind vier Umlenkzapfen angeordnet, über die der obere und untere Strang 51, 52 der Befestigungsschnur 5 geführt werden.

Das Befestigungsmittel 6 ist als paariger Dauermagnet ausgebildet. Der äußere, zapfenförmigen Teil des Befestigungsmittels 6 ragt form- und kraftschlüssig in den nicht näher dargestellten, unteren, topfförmig ausgebildeten Dauermagneten ein. Durch eine konische Gestaltung des Befestigungsmittels 6 wirkt der Verschluss selbstzentrierend.

**Figur 2** zeigt die im befestigten Zustand dem Helm zugewandte Seite einer alternativen Ausgestaltung des Befestigungselements 6 mit einem besonders einfachen Aufbau. Das Befestigungselement 6 hat drei Haltestege 17 für die Befestigungsschnüre 5, mit denen der Benutzer die Länge der Schnüre 5 variieren und damit an seine Bedürfnisse anpassen kann. Die Befestigung am Helm erfolgt wie oben beschrieben über einen Dauermagneten 18, der begrenzt beweglich in einer Einfassung 19 angeordnet ist. Der Dauermagnet 18 hat mittig eine Erhöhung 20 in Form eines Kreiszylinderstumpfes. Am Helm ist, in der Figur 2 nicht ersichtlich, als Gegenstück ebenfalls ein Dauermagnet in der Helmschale angeordnet. Durch die Ausgestaltung der Dauermagneten 18 mit der mittigen Erhöhung 20 wird eine sichere Befestigung gewährleistet, da die Erhöhung/ Vertiefung einem Zug in Richtung der Helmschale entgegenwirkt. Zum anderen wird die Lage der beiden Magneten zueinander bei der Befestigung selbstständig zentriert, so dass ein präziser sicherer Halt der Brille gewährleistet wird. So kann die Brille auch mit Handschuhen einfach befestigt werden, indem die Befestigungsmittel nur in die Nähe der in der Helmschale befindlichen Dauermagneten gebracht werden müssen und sich dort selbstständig verbinden und zentrieren.

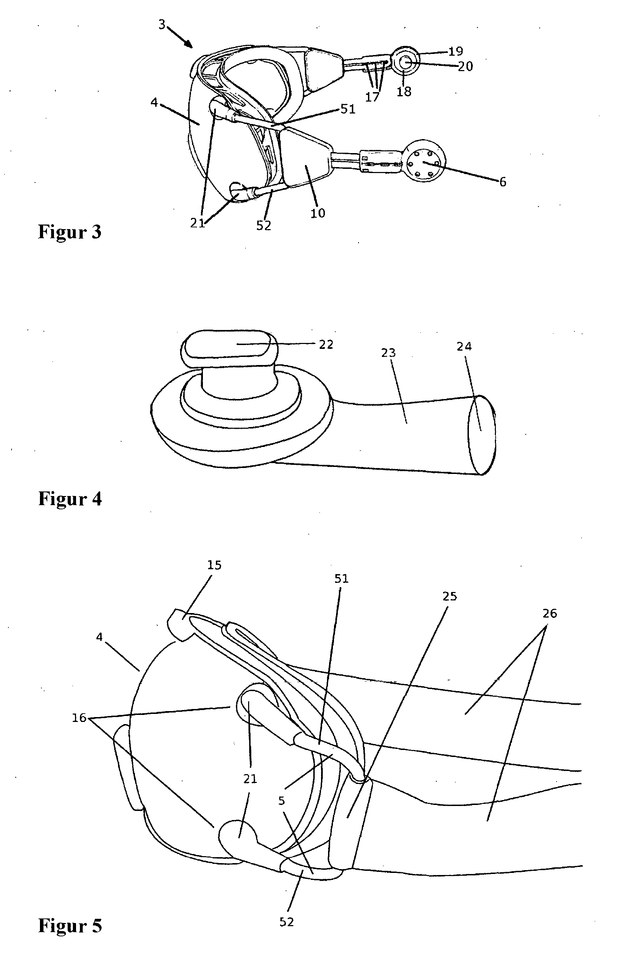

**Figur 3** zeigt eine Brille 3 mit einem Befestigungselement entsprechend der alternativen Ausgestaltung nach Figur 2 mit einem Spannelement 10 und Scheibenverbindern 21. Die Scheibenverbinder 21 sind so am Rand der Sichtscheibe 4 angeordnet, dass die Fortsätze über die Kante der Sichtscheibe hinausragen. Dadurch werden die Befestigungsschnüre 51 und 52 nicht durch Reibung an der Kante der Sichtscheibe 4 beschädigt. Durch das Spannelement 10 kann der Benutzer die Andruckkraft der Brille 3 an das Gesicht regulieren. Durch die Einstellmöglichkeit der Länge der Befestigungsschnüre 5 über die Haltestege 17 im Befestigungselement 6 (Figur 2) in Verbindung mit dem Spannelement 10 bietet die Brille gute Einstellbarkeit an die individuelle Gesichtskontur.

**Figur 4** zeigt einen Scheibenverbinder 21 mit dem die Sichtscheibe (4) oder die Sichtschiebensegmente oder Sichtgläser durch Öffnungen in der Sichtscheibe hindurch im Rahmen 15 befestigt sind. Der Scheibenverbinder weist zum Zwecke dieser Befestigung eine Renkverbindung 22 auf sowie einen Fortsatz 23 an dem die Befestigungsschnüre 5 angebracht sind. Der Fortsatz 23 ist rohrförmig ausgebildet und kann so die Befestigungsschnüre 5 aufnehmen. Die Befestigungsschnüre werden durch eine Einschuböffnung 24 in den Scheibenverbinder 21 geschoben und im einfachsten Fall gesichert indem das an der anderen Seite austretende Ende verknotet wird. Das dem Fortsatz 23 gegenüberliegende Ende des Scheibenverbinders 21 ist um ca. 90° abgewinkelt, wobei der konkrete Winkel von der Ausgestaltung der Sichtscheibe abhängt, und weist dort die Mittel für die Renkverbindung auf. Zu dieser Renkverbindung 22 hin verdickt sich der Durchmesser des rohrförmigen Fortsatzes 23 etwas. Die Verbindung zwischen den Befestigungsschnüren und dem Rahmen erfolgt mittels der Renkverbindung 22 Der Fortsatz 23 des Scheibenverbinders ragt über die Kante der Sichtscheibe hinaus. Dadurch werden die Befestigungsschnüre derart geführt, dass sie nicht mit der Kante der Sichtscheibe in Berührung kommen. Darüber hinaus wirkt der Fortsatz 22 des Scheibenverbinders 21 als Hebel und der die Brille mit gleichem Anpressdruck an das Gesicht des Trägers andrückt. Um auch in Verbindungsrichtung ein Herausgleiten der Renkverbindung 22 zu verhindern, sind die existierenden Teile nicht exakt formschlüssig mit den im Rahmen oder in der Sichtscheibe befindlichen, korrespondierenden Aufnahmeöffnungen 24 Die Renkverbindung 22 muss daher auch in Verbindungsrichtung mit leichtem Druck in die dafür vorgesehenen Öffnungen eingeschnappt werden. Zur verschleißfreien Anwendung ist die Renk- und Schnappverbindung 22 dafür ausreichend elastisch gestaltet.

**Figur 5** zeigt eine Sportbrille 3 mit Rahmen 15. Am Rahmen 15 sind im Randbereich beidseitig schlaufenförmige Befestigungsschnüre 5 an zwei Anlagepunkten 16 mittels Scheibenverbinder 21 in der Sichtscheibe 4 angelenkt. Die Scheibenverbinder 21 sind mittels Renkverbindung (Bajonettverbindung) in der Sichtscheibe befestigt. Dabei ist sowohl am oberen Strang 51 der Befestigungsschnur 5 als auch am unteren Strang 52 der Befestigungsschnur 5 jeweils ein Scheibenverbinder 21 angeordnet. Die Scheibenverbinder 21 am Ende der beiden oberen Stränge 51 der Befestigungsschnüre 5 sind dabei im oberen seitlichen Bereich der Sichtscheibe befestigt und die Scheibenverbinder 21 an den Enden der beiden unteren Strängen 52 der Befestigungsschnüre 5 entsprechend im unteren seitlichen Bereich der Sichtscheibe 4. Dadurch wird die freie Sicht des Benutzers durch die Scheibenverbinder 21 nicht eingeschränkt.

Die Scheibenverbinder 21 sind wie oben beschrieben mittels Renkverbindung 22 (Bajonettverbindung) in der Sichtscheibe 4 befestigt, wobei diese zur Mitte der Sichtscheibe 4 gerichtet eingesetzt werden und dann durch die Drehung in Richtung der Scheibenaußenseite in der Sichtscheibe 4 befestigt sind. Die Scheibenverbinder 21 sind somit bei der Benutzung drehbar und passen sich dadurch an das Gesicht und die Kopfform des Trägers an. Von der Befestigung in der Sichtscheibe 4 setzt sich der Scheibenverbinder 21 vom Anlagepunkt 16 noch ca. 2 cm beabstandet und weitestgehend parallel zu Sichtscheibe fort. Das andere, schlaufenförmige Ende der Befestigungsschnur 5 steht mit einem dehnbaren Befestigungselement 26 insbesondere einem Gummiband, in Verbindung. Dieses ist um einen Sporthelm 2 oder den Kopf des Trägers umlegbar und erzeugt mittels Kontraktion einen Anpressdruck der die Sportbrille 3 an diesem befestigt.

Das dehnbare Befestigungselement 26 weist einen geschlossenen Kanal 25 auf, der die schlaufenförmige Befestigungsschnur 5 aufnimmt. Dieser Kanal 25 befindet sich in dem schlaufenförmigen Ende des dehnbaren Befestigungselements 26.

### Bezugszeichenliste:

- 2: Sporthelm
- 3: Sportbrille
- 4: Sichtscheibe
- 5: Befestigungsschnur
- 51: oberer Strang der Befestigungsschnur 5
- 52: unterer Strang der Befestigungsschnur 5
- 6: Befestigungsmittel/ Befestigungselement
- 7: Nasenaussparung
- 8: Löcher
- 10: Spannelement
- 13: Spannhebel
- 14: Drehachse des Schwenkhebels
- 15: Rahmen der Sportbrille
- 16: Anlagepunkt
- 17: Haltesteg
- 18: Dauermagnet
- 19: Einfassung
- 20: Erhöhung
- 21: Scheibenverbinder
- 22: Renkverbindung
- 23: Fortsatz
- 24: Einschuböffnung
- 25: Kanal
- 26: dehnbares Befestigungselement

## Patentansprüche

1. Sportbrille (3) aufweisend einen Rahmen (15) zur Aufnahme einer Sichtscheibe (4) oder mehrerer Sichtscheibensegmente oder Sichtgläser, wobei in den seitlichen Bereichen jeweils mindestens ein, vorzugsweise zwei Scheibenverbinder (21) mittels Renkverbindung durch Öffnungen in der Sichtscheibe (4) bzw. den Sichtscheibensegmenten hindurch im Rahmen (15) befestigt sind, wodurch die Sichtscheibe (4) bzw. die Sichtscheibensegmente an den Rahmen (15) angelegt werden, wobei die Scheibenverbinder (21) jeder Seite mit mindestens einer biegeschlaffen, elastischen Befestigungsschnüre (5) verbunden sind, welche jeweils mit Befestigungsmitteln (6) in Verbindung steht.

2. Sportbrille (3) nach Anspruch 1, **dadurch gekennzeichnet, dass** die Befestigungsmittel und Befestigungselemente als paarweise Dauermagneten ausgeführt sind, wobei die Befestigungsmittel im Seitenbereich der Helmschale, vorzugsweise im Schläfenbereich angeordnet sind und eine lösbare, vorzugsweise kraft- und/oder formschlüssige Verbindung von Sportbrille (3) und Sporthelm (2) ermöglichen.

3. Sportbrille (3) nach einem der vorangegangenen Ansprüche, **dadurch gekennzeichnet, dass** die ausreichend elastischen Scheibenverbindern (21) mit dem Rahmen (15) mittels einer Renk- und Schnappverbindung (22) verbunden sind, derart dass die Renk- und Schnappverbindung (22) in Verbindungsrichtung nicht exakt formidentisch ist und nur mittels leichtem Druck geschlossen werden kann.

4. Sportbrille (3) nach einem der vorangegangenen Ansprüche, **dadurch gekennzeichnet, dass** die Sichtscheibe (4) aus einer oder mehreren Sichtscheibensegmenten besteht, wobei an die Innenseite eines Sichtscheibensegments andere Sichtschiebensegmente beabstandet aufgebracht sind oder diese durch die Verbindung mit dem Rahmen (15) zusammengefügt sind.

5. Sportbrille (3) nach einem der vorangegangenen Ansprüche, **dadurch gekennzeichnet, dass** die Befestigungsmittel (6) und Befestigungselemente als paarweise Dauermagneten (18) ausgebildet sind.

6. Sportbrille (3) nach einem der vorangegangenen Ansprüche, **dadurch gekennzeichnet, dass** mit den Befestigungsschnüren (5) ein dehnbares Befestigungselement in Verbindung steht, das um einen Sporthelm (2) oder den Kopf des Trägers umlegbar ist, wobei es sich an diesen anpresst und die so die Sportbrille (3) mit diesem verbindet.

7. Sportbrille (3) nach Anspruch 1 oder 2, **dadurch gekennzeichnet, dass** die Befestigungsschnüre (5) aus elastischer Rundlitze, runder Gummilitze, Rundgummi, Gummikordel oder Gummigarn mit Polyester- oder Polyamid-Fasern oder Neoprenband bestehen.

8. Sportbrille (3) nach einem der Ansprüche 1 bis 3, **dadurch gekennzeichnet, dass** ein rahmen- oder hülsenförmiges Spannelement (10) die beabstandeten Befestigungsschnüre (5) umgreift und auf ihnen axial verschiebbar ist.

## Claims

1. Sports glasses (3) containing a frame (15) to accommodate a visor (4) or several visor segments or an eye shield, whereby in the lateral areas there are at least one, and preferably two, visor connectors (21) fastened by means of a bayonet connection through openings in the visor (4) respectively through the visor segments in the frame (15), whereby the visor (4), respectively the visor segments, are put against the frame (15), whereby the visor connectors (21) of each side are connected with at least one soft, elastic attachment cord (5), which cord is connected in its turn with attachment units (6).

2. Sports glasses (3) according to claim 1, **characterized in that** the attachment units and attachment elements are paired permanent magnets, whereby the attachment units are arranged in the lateral area of the helmet shell, preferably around the temples, and allow a detachable, preferably friction locking and/or form-fit connection between the sports glasses (3) and the sports helmet (2).

3. Sports glasses (3) according to any of the previous claims, **characterized in that** the sufficiently elastic visor connectors (21) are connected to the frame (15) by means of a bayonet and snap connection (22) in such a manner that the bayonet and snap connection (22) is not exactly identical in form in the direction of connection and may only be locked if slight pressure is applied.

4. Sports glasses (3) according to any of the previous claims, **characterized in that** the visor (4) consists of one or several visor segments, whereby to the inner side of one visor segment, other visor segments are arranged, spaced apart, or whereby these are joined together through connection with the frame (15).

5. Sports glasses (3) according to any of the previous claims, **characterized in that** the attachment units (6) and attachment elements are paired permanent magnets (18).

6. Sports glasses (3) according to any of the previous claims, **characterized in that** an elastic attachment element is connected to the attachment cords (5), which may be put round a sports helmet (2) or the head of its carrier, pressing itself against said helmet or head; and thus connecting the sports glasses (3) to it.

7. Sports glasses (3) according to claim 1 or 2, **characterized in that** the attachment cords (5) are made of elastic round braid, elastic ribbon, elastic cord or elastic thread with polyester or polyamide fibres, or neoprene band.

8. Sports glasses (3) according to any of the claims 1 to 3, **characterized in** a frame- or sleeve-shaped pulling element (10) clasps the spaced-apart attachment cords (5), and may be moved along them in an axial direction.

## Revendications

1. Paire de lunettes de sport (3) présentant une monture (15) destinée à recevoir une visière (4) ou plusieurs segments de visière ou verres de vision, sachant que dans chacune des régions latérales, au moins une et de préférence deux attaches de visière (21) sont fixées au moyen d'un assemblage à baïonnette dans la monture (15) en traversant des ouvertures dans la visière (4) ou dans les segments de visière, de sorte que la visière (4) ou les segments de visière sont appliqués contre la monture (15), sachant que les attaches de visière (21) de chaque côté sont reliées à au moins un cordon de fixation élastique (5), mou en flexion, qui est respectivement relié à des moyens de fixation (6).

2. Paire de lunettes de sport (3) selon la revendication 1, **caractérisée en ce que** les moyens de fixation et éléments de fixation sont réalisés sous la forme de paires d'aimants permanents, sachant que les moyens de fixation sont disposés dans la région latérale de la coque du casque, de préférence dans la région des tempes, et permettent une liaison amovible, de préférence à force et/ou par complémentarité de forme, entre la paire de lunettes de sport (3) et le casque de sport (2).

3. Paire de lunettes de sport (3) selon l'une des revendications précédentes, **caractérisée en ce que** les attaches de visière (21), suffisamment élastiques, sont reliées à la monture (15) au moyen d'un assemblage (22) à baïonnette ou par enclenchement, de telle sorte que l'assemblage (22) à baïonnette ou par enclenchement n'est pas de forme exactement identique dans la direction d'assemblage et ne peut être fermé qu'au moyen d'une légère pression.

4. Paire de lunettes de sport (3) selon l'une des revendications précédentes, **caractérisée en ce que** la visière (4) est constituée d'un ou plusieurs segments de visière, sachant que d'autres segments de visière sont installés à distance sur le côté intérieur d'un segment de visière, ou que ces segments de visière sont assemblés entre eux par l'assemblage avec la monture (15).

5. Paire de lunettes de sport (3) selon l'une des revendications précédentes, **caractérisée en ce que** les moyens de fixation (6) et éléments de fixation sont réalisés sous la forme de paires d'aimants permanents (18).

6. Paire de lunettes de sport (3) selon l'une des revendications précédentes, **caractérisée en ce qu'**un élément de fixation extensible est relié aux cordons de fixation (5), élément qui peut être posé autour d'un casque de sport (2) ou de la tête de l'utilisateur, sachant qu'il s'applique contre le casque ou la tête et relie ainsi la paire de lunettes de sport (3) au casque ou à la tête.

7. Paire de lunettes de sport (3) selon la revendication 1 ou 2, **caractérisée en ce que** les cordons de fixation (5) sont constitués de fil toronné rond élastique, de fil toronné rond en caoutchouc, de caoutchouc rond, de cordon en caoutchouc ou de fil en caoutchouc avec des fibres de polyester ou de polyamide, ou d'une bande de néoprène.

8. Paire de lunettes de sport (3) selon l'une des revendications 1 à 3, **caractérisée en ce qu'**un élément tendeur (10) en forme de cadre ou de manchon s'engage autour des cordons de fixation mutuellement distants (5) et peut être déplacé axialement sur ceux-ci.
